# EUROPEAN PATENT APPLICATION

(11) **EP 1 382 614 A1**
(43) Date of publication of application: **21.01.2004**
(21) Application number: 02015589.1
(22) Date of filing: 15.07.2002
(51) Int. Cl.: C07K 14/54

(54) **Process for the purification of interleukin-4 and its muteins**

(71) Applicant: Bayer HealthCare AG, 51368 Leverkusen (DE)
(72) Inventor: Peters, Jörg, Dr., 42781 Haan (DE); Minuth, Torsten, Dr., 42109 Wuppertal (DE); Dellweg, Hans-Georg, Dr., 42113 Wuppertal (DE)

(57) **Abstract**

This invention relates generally to a method for purifying Interleukin-4 or related variants from an *Escherichia coli* culture medium, where Interleukin-4 and its derivatives are expressed as insoluble protein aggregates, so-called inclusion bodies.

## Description

### Background of the Invention

### Field of the Invention

This invention relates generally to a method for purifying Interleukin-4 or related variants from an *Escherichia coli* culture medium, where Interleukin-4 and its derivatives are expressed as insoluble protein aggregates, so-called inclusion bodies.

### Description of Related Art

Human Interleukin-4 (hIL-4) is a 15.000 dalton polypeptide with a basic pI of about 10.5 and murine IL-4 (mIL-4) is a 13.600 dalton polypeptide with a neutral pI of 6.5. Interleukin-4 belongs to a family of cytokines inducing and coordinating the proliferation, the maturation, the survival and the differentiation of lymphoid and myeloid cells (Callard R, Gearing A (1994): The cytokine facts book. Academic Press, London, San Diego). Complete antagonists, partial and selective agonists of hIL-4 and mIL-4 have been described by Sebald and Grunewald et al. (Sebald W (1998): United States Patent 5,723,118 (WO93/10235). Date of patent 3 March, 1998; Grunewald SM, Kunzmann S, Schnarr B, Ezernieks J, Sebald W, Duschl A (1997): The Journal of Biological Chemistry 272(3): 1480-1483; Shanafelt A et al. (1997): US-Patent WO97/47744. Date of patent 14 June, 1996). Muteins of human IL-4 leading to improved folding yields have been described recently (Domingues H, Peters J, Schneider KH, Apeler H, Sebald W, Oschkinat H, Serrano L (2000): J. Biotechnol. 84: 217-230).

The cDNA of hIL-4 has been expressed in E. *coli* as insoluble inclusion bodies (Kastelein RA, van Kimmenade A (1987): European patent application EP 0301835/A1, Date of patent 29 July, 1987; van Kimmenade A, Bond MW, Schumacher JH, Laquoi C, Kastelein RA (1988) Eur. J. Biochem. 173: 109-114; Jayaram B, Bevos R, Guisez Y, Fiers W (1989) Gene 79: 345-354). It has been shown that recombinant human IL-4 possessing a high level of biological activity can be isolated from this source. However, expression rates, overall purification yields and refolding yields are not sufficient for commercial use of these systems.

Clinical use of active human IL-4 or related variants requires a high purity material that is not contaminated by cell constituents or contaminants of the respective expression system. Accordingly, there is a need for purification of active IL-4 or related muteins from optimized *E. coli* expression systems (Apeler H, Wehlmann H (1999) Plasmids, their construction and their use in the manufacture of Interleukin-4 and Interleukin-4 muteins. European patent application EP 00100129.6. Date of patent 7 January, 1999) in high yields and high purity.

The production of large quantities of biologically active polypeptides with human origin by recombinant DNA techniques has become the method of choice, since heterologous proteins can be expressed in sufficient amounts in recombinant bacteria or other host cells.

This invention relates to purifying insoluble inclusion bodies containing high amounts of IL-4 or its variants and further purifying the solubilized molecule to a correctly folded, biologically active, highly pure polypeptide.

Genetically engineered bio-pharmaceuticals are typically purified from the cell-free culture supernatant containing a variety of host cell contaminants like, e.g., nucleic acids, host cell proteins, endoxotins, lipids, polysaccharides etc.. There is a need in the art for an efficient method for selectively purifying Interleukin-4 or its muteins from other molecules, particularly from those which are very closely related and difficult to separate like disulfide-isoforms, derivatives containing amino acid mismatches, oxidized forms and, formylated molecules.

Purification of Interleukin-4 from *E. coli* cells expressing the target protein in an aggregated insoluble form, so-called "inclusion bodies", was first described by Kastelein and van Kimmenade (Kastelein RA, van Kimmenade A (1987): European patent application EP 0301835/A1, Date of patent 29 July, 1987). Cells derived from low cell density fermentation were disrupted by sonication and, after centrifugation, the remaining pellet was dissolved in 5 M guanidinium-HCl containing oxidized and reduced glutathione. Refolding was accomplished by 10-fold dilution to a final protein concentration of 250 mg/L. The precipitate formed was then removed by centrifugation and the supernatant was again dialyzed against phosphate buffered saline. Again centrifugation was necessary to remove the novel precipitate. Then, ultrafiltration was used to concentrate the protein to 8 g/L, thereby again forming precipitates. Finally, the refolded and concentrated Interleukin-4 was chromatographed only on a size exclusion resin. Assays for determination of the biological activity of human Interleukin-4 have also been described in the cited article. Clearly, refolding and subsequent steps suffer from heavy precipitation and the chromatography steps involved can not separate closely related isoforms. Furthermore, the purified Interleukin-4 was contaminated by oxidized species and other related products. Van Kimmenade and coworkers did not give figures with regard to the endotoxin and host cell protein content of the final product. However, it is common knowledge that ultrafiltration and gel filtration do not lead to high clearance factors with respect to nucleic acids, endotoxins and host cell proteins. Hence, the product can not be expected to yield pharmaceutically acceptable quality.

Murine IL-4 has been isolated from *Escherichia coli* from inclusion bodies by Levine and coworkers (Levine AD, Rangwala SH, Horn NA, Peel MA, Matthews BK, Leimgruber RM, Manning LA, Bishop BF, Olins PO (1995): J. Biol. Chem. 270(13): 7445-7452). The method involves a refolding step and two subsequent chromatographic steps (cation exchange, gel filtration). However, the murine IL-4 contained an N-terminal extension as well as different isoforms. The removal of host cell impurities like endotoxins or nucleic acids was not described by Levine and coworkers. Hence, the method is not able to sufficiently discriminate between these contaminants and is not applicable to the purification of pharmaceutical grade human IL-4 or its muteins from *E. coli* inclusion bodies.

Accordingly, it is an objective of this invention to provide an improved process for isolation of non-native Interleukin-4 or its muteins, efficiently refolding Interleukin-4 or its muteins to form disulfide bridges and to separating Interleukin-4 from its closely related isoforms, derivatives and host cell contaminants by means of chromatography and filtration.

It is still object to provide a process for purifying Interleukin-4 that results in considerable improvement in its homogeneity.

These and other objects will be apparent to those of ordinary skill in the art.

### Summary of the Invention

The invention provides, in one aspect, a process for effectively disrupting *E. coli* cells and, subsequently, for purifying the released aggregated Interleukin-4 or its muteins by a combination of centrifugation and cross-flow microfiltration, resulting in a highly pure inclusion body preparation which can be used as starting material for effectively refolding of Interleukin-4 to its native structure.

In another aspect, the invention provides a process for renaturation of Interleukin-4 or its muteins at relatively high protein concentrations giving a high refolding yield. Recovery of protein is generally more than 90%.

The isolation of recombinant Interleukin-4 involves separation of the protein from a variety of different host cell contaminants. Each step of the process involves special buffers that enable sufficient separation to take place. The presence of several Interleukin-4 variants, that normally co-purify using conventional chromatographic media, necessitates special final chromatography steps and conditions. These species primarily consist of disulfide-conformers of Interleukin-4 or its muteins, methionine-sulfoxide variants, variants with partially clipped formyl-methionine residues at the N-terminus, variants with mis-incorporated amino acids and, all kinds of permutations of these variations which are possible.

In the present invention, conditions are identified for favorably separating Interleukin-4 or its muteins from these and other closely related variants. Using this process, recombinant Interleukin-4 and its muteins can be isolated in high purity and commercially attractive yields.

### Detailed description

### A. Definitions

As used herein, "IL-4" refers to Interleukin-4 from any species, including bovine, ovine, porcine, equine, avian, mouse, rat, and preferably human, in native sequence or in variant form, and from any source, whether natural, synthetic, or recombinantly produced. Preferably, the IL-4 is produced recombinantly. In a preferred method, the IL-4 is cloned and its DNA expressed, e.g., by the process described in Apeler & Wehlmann, 1999 (Apeler H, Wehlmann H (1999) Plasmids, their construction and their use in the manufacture of Interleukin-4 and Interleukin-4 muteins. European patent application EP 00100129.6. Date of patent 7 January, 1999.

The preferred IL-4 variants are those described in U.S. Patent 5,723,118 (WO93/10235) issued March 3, 1998.

As used herein, "buffer" refers to a buffered solution that resists changes in pH by the action of its acid-base conjugate components. The buffer for the refolding aspect of this invention has a pH range of about 6-9, preferably 6.5-8. Examples of buffers that will control the pH within this range are citrate-NaOH, GOOD-buffers like TRIS-HCl or Triethanoamine-HCl, and, preferably, phosphate buffers.

The buffer for the ceramic hydroxyapatite chromatography aspect of this invention has a pH range of about 5-9, preferably 6-8. Examples of buffers that will control the pH within this range are preferably phosphate buffers. If elution is not performed by a linear increase of phosphate concentration, a salt, preferably NaCl or KCl, is added to the buffer in a concentration ranging from 5 mM up to the solubility limit of the salt.
The buffer for the metal chelate affinity chromatography aspect of this invention has a pH range of 6-9, preferably 7-8. Examples of buffers that will control the pH within this range are phosphate and other non-chelating components (so-called non-GOODs buffers like e.g. glycine-NaOH, N-ethylmorpholino-HCl or 2-amino-2-methyl-1,3-propandiol-HCl.

As used herein, "modifier reagents" refers to an ion pairing reagent used for reversed phase chromatography like, for example, trifluoracetic acid, ammonium salts, phosphate salts, acetic acid, and NaCl.

As used herein, the phrase "phosphate salt" refers to a salt having a cation, preferably from the alkaline earth or alkali metal elements or an ammonium cation, and having a phosphate anion. Sxamples of such salts include sodium phosphate, calcium phosphate, ammonium phosphate, magnesium phosphate, and potassium phosphate. The most preferred salts herein are sodium and potassium phosphate.

As used herein, "alcohols" and "alcoholic solvents" are meant in the sense of the commonly used terminology for alcohol, preferably alcohols with 1 to 10 carbon atoms, more preferably methanol, ethanol, iso-propanol, n- or t-propanol, as well as glycerol, propylene glycol, ethylene glycol, polypropylene glycol, and polyethylene glycol, and most preferably ethanol or iso-propanol. Such solvents are solvents that, when added to aqueous solution, increase the hydrophobicity of the solution by decreasing solution polarity.

"Polar aprotic solvents" are such molecules as dimethyl sulfoxide (DMSO), dimethyl formamide (DMF), N-methylpyrrolidone (NMP), tetrahydrofurane (THF), dioxane, acetonitrile (ACN), etc., that can be used in place of or in addition to the alcohol. The most preferred polar aprotic solvent herein is acetonitrile.

### B. Modes for Carrying Out the Invention

The process herein involves first, purifying of non-native Interleukin-4 from procaryotic host cells in a suitable isolation buffer by any appropriate technique as insoluble aggregates, so-called "inclusion bodies". Such appropriate techniques are, e.g., exposure of the cells to a buffer of suitable ionic strength to solubilize most host proteins, but in which aggregated Interleukin-4 is substancially insoluble, and disrupting the cells so as to release the inclusion bodies and make them available for recovery by, for example, centrifugation or microfiltration. This technique is well known, and is described, for example, in U.S. Patent 4,511,503.

Briefly, the cells are suspended in the buffer (typically at pH 5-9, preferably at pH 6-8, using an ionic strength of about 0.01 to 2 M, preferably 0.1 to 0.2 M). Any suitable salt, including sodium chloride, is useful to maintain a sufficient ionic strength value. The cells, while suspended in this buffer, are then disrupted by lysis using techniques commonly empolyed such as, for example, mechanical methods, e.g. a Manton-Gaulin press, French press, Bran & Lübbe homogenizer or microfluidizer, or a sonic oscillator, or by chemical or enzymatic methods employing lysozyme, or by any combination of these methods.

After the cells are disrupted, the suspension is typically centrifuged to pellet the inclusion bodies. In one embodiment, this step is carried out at about 500 to 15.000 x g, preferably about 12.000 x g, in a standard centrifuge for a sufficient time that depends on volume and centrifuge design, usually about 10 to 30 minutes. The resulting pellet contains substancially all of the inclusion bodies, but in case the disruption was not complete, also cell wall fragments and unbroken cells are sedimented. Completeness of cell disruption can be assayed by resuspending the pellet in a small volume of disruption buffer and assaying this suspension with a phase-contrast microscope, by laser diffraction (e.g. Mastersizer 2000), or by determining the total soluble protein by means of dye-methods according to well known methods (e.g. Bradford et al. (1976): Anal. Biochem. 72: 248-254). The presence of broken cells or whole cells or the incompleteness of released soluble protein indicates that additional disruption is necessary. The suspension is again centrifuged and the pellet recovered, resuspended, and analyzed.

The pH range of a suitable inclusion body washing buffer typically is between pH 7-10, preferably pH 8-9. Examples of buffers that will provide a pH within this latter range include glycine, CAPSO (3-[Cyclohexylamino]-2-hydroxy-1-propane-sulfonic acid), AMP (2-Amino-2-methyl-1 -propanol), CAPS (3-[Cyclohexylamino]-1-propane-sulfonic acid), CHES (2-[N-Cyclohexylamino]-ethane sulfonic acid) and TRIS-HCL (Tris-[hydroxymethyl]aminomethane hydrochloride). The preferred buffer herein is TRIS-HC1, preferably at concentrations of 50 to 500 mM, more preferably at 100 to 200 mM, at a pH of about 7-10, preferably about 8-9. Additional substances included in the washing buffer are chelating substances like, e.g., ethylenediamintetraacetic acid (EDTA), ethyleneglycol-O,O'-bis-(2-aminoethyl)-N,N,N',N'-tetraacetic acid (EGTA), nitriloacetic acid (NTA) or trans-1,2-diamino-cyclohexan-N,N,N',N'-tetraacetic acid (CDTA) complexing Ca²⁺-ions which are know to bridge lipopolysaccharides (LPS) in the outer cell wall of *E. coli* (Witholt et al. (1976): Biochim. Biophys. Acta 443(3): 534-544). Furthermore, a suitable detergent, which effectively solubilizes lipids bound to the surface of the inclusion body or lipids contained in cell wall fragements, like non-ionic detergents (e.g. Triton X-100, Tween-series, dodecylmaltoside etc.), ionic surfactants (e.g. SDS, CTAC, CTAB, cholic acid and its derivatives) or, most preferably, zwitterionic detergents (e.g. Zwittergent-series, CHAPS, CHAPSO, desoxycholate) should be included in the washing buffer. The concentration of these surfactants should be sufficiently high to ensure the formation of detergent-micelles and mixed detergent-lipid-micelles and depends on the critical micellar concentration (CMC) of the respective surfactant. Preferably, the concentration of the surfactant in the washing buffer should be well above, most preferably 2-fold above, the CMC-value. Most preferably, Zwittergent 3-14 can be used at concentrations between 0.01% to 5%, preferably between 0.1% to 1%, to effectively solubilize lipids associated with inclusion bodies or cell wall fragments. Additionally, salts like e.g. NaCl, KCl, Na₂SO₄ may be included in the washing buffer to increase the effectiveness of the removal of outer membrane proteins, although this may result in a loss of Interleukin-4 recovery (10-40% loss) depending on the detergent utilized. Furthermore, mercaptans like e.g. dithiothreitol, mercaptoethanol or cysteine may be included in the washing buffer at concentrations of 0.1 to 10 mM, preferably at 0.1 to 1 mM, to reduce any covalent cystine bridges present in the inclusion body. Nucleic acids may represent up to 0.5% of the protein content in inclusion bodies (Valax & Georgiou (1993): Biotechnol. Prog. 9(5): 539-547) and therefore, RNAse or, preferably, DNAse may be included in the washing buffer. Other chaotropic substances like e.g. urea at concentrations of 0.5 to 8 M, preferably 2 to 4 M, or guanidinium hydrochloride at concentrations of 0.5 to 4 M, preferably 2 to 4 M, may also be added to the washing buffer.

Additionally, further purification of the inclusion bodies by cross-flow microfiltration may be necessary to remove cell debris with a density in the range of the inclusion bodies as well as soluble host cell impurities such as endotoxins, nucleic acids, proteins or low molecular weight contaminants. For this purpose, membranes with exclusion limits of 0.1 µm to 0.65 µm, preferably 0.22 and 0.45 µm, most preferably 0.45 µm can be used. Suitable membrane materials include polysulfone and polyethersulfone membranes with or without surface modifications, teflon (PVDF) membranes, Nylon membranes, PTFE membranes, and, preferably, cellulose or cellulose acetate membranes and their derivatives. Suitable devices include hollow fibre systems with straight or coiled fibres, dynamic membrane filtration units, where a membrane rotates to minimize the development of a membrane layer, vibration membrane filtration units, where the formation of a membrane layer is minimized by the influence of vibration, cassette systems without a screen (open channel), and, most preferably, cassette systems equipped with screens of different types of design. For example, a Hydrosart super wide channel cassette with a cut-off of 0.45 µm can be used (Patent application PCT/01/04634, german patent application DE 100 22 258 A1). The washing buffer used for cross-flow microfiltration is the same as mentioned above.

After harvesting the washed inclusion body pellet from the centrifuge or from the cross-flow microfiltration system, the material can be stored over extended periods of time by freezing in liquid gas, e.g. liquid nitrogen or carbondioxide. For this purpose, several techniques are commercially available (e.g., Messer Griesheim, Krefeld, Germany; Integrated Biosystems, Benice, USA). The inclusion body suspension is pumped with a constant stream into a stirred vessel containing either liquid nitrogen or liquid carbondioxide. Upon contact of the inclusion body suspension with the liquid gas the droplet immediately freezes. Ultimately, the frozen inclusion body pellets can be removed from the vessel by a sieve and stored at a temperature below the eutectic point.

Once obtained from the cryopellet system, the Interleukin-4 is suitably refolded into an active conformation as described below.

The inclusion body cryopellets containing non-native Interleukin-4 are thawn rapidly and the inclusion bodies are then solubilized in alkaline buffer by the addition of a suitable anionic or cationic detergent or a chaotropic salt like, e.g., urea, KSCN, NaJ, MgCl₂ or, most preferably, guanidinium salts. Examples for detergents are, e.g., sodium dodecylsulfate, sodium tetradecylsulfate, laurylsarcosine, cholic acid and its derivatives, taurocholic acid and its derivatives and tert-octyl phenyl propanesulfonic acid (TOPPS) (anionic surfactants) or cetyltrimethylammonium chloride or bromide, hexadecyltrimethylammonium bromide and tetradecyltrimethylammonium bromide (cationic surfactants), which effectively solubilize inclusion bodies at low concentrations of 0.1% to 10%, preferably 0.5%. The incubation takes place under conditions of inclusion body concentration, incubation time, and incubation temperature that will allow solubilization of the Interleukin-4 to occur in the alkaline buffer.

Measurement of the degree of solubilization of the Interleukin-4 in the buffer is suitably carried out by turbidity determination, by analyzing Interleukin-4 fractionation between the supernatant and pellet after high speed centrifugation (12.000 x g, 30 min) on SDS-PAGE, by protein assay (BCA-assay (Pierce)), or by RP-HPLC.

The pH range of the alkaline buffer for solubilization typically is at least about 8, with the preferred range being about 7-10. Examples of buffers that will provide a pH within this latter range include glycine, CAPSO (3-[Cyclohexylamino]-2-hydroxy-1-propane-sulfonic acid), AMP (2-Amino-2-methyl-1-propanol), CAPS (3-[Cyclohexylamino]-1-propane-sulfonic acid), CHES (2-[N-Cyclohexylamino]-ethane sulfonic acid) and TRIS-HCL (Tris-[hydroxymethyl]aminomethane hydrochloride). The preferred buffer herein is TRIS-HCl, preferably at concentrations of 200 mM, at a pH of about 7-10, preferably about 8-9.

The concentration of Interleukin-4 in the buffered solution for solubilization must be such that the Interleukin-4 will be substancially solubilized and fully denatured. It is desirable to produce a more concentrated solubilized protein solution prior to refolding in order to keep the residual concentrations of the chaotropic agent or the detergent as low as possible. Thus, the preferred concentration of Interleukin-4 is at least 10 g/L, with a more preferred range of 20-30 g/L. After solubilization, the Cysteine-SH groups may be either reduced by the addition of a sulhydryl-reducing agent like mercaptoethanol, dithiothreitol (DTT) or L-cysteine, or chemically derivatized by the addition of sulfite and an oxidizing agent like, e.g., iodosobenzoate or, preferably, tetrathionate (Kella & Kinsella (1985): J. Biochem. Biophys. Methods 11:251-263; Kella et al. (1988): J. Prot. Chem. 7: 535-548).

For example, Interleukin-4 may be solubilized in 8 M guanidinium hydrochloride to a concentration of 10 g/L, sulfitolyzed by the addition of sulfite and tetrathionate in 50 and 100 fold molar excess, dialyzed or diafiltered against the solubilization buffer containing 4 M guanidinium hydrochloride to remove excess derivatization reagents, diluted to, for example, 50 mg/L for folding. For dialysis, bags with a cut-off of 3500 Dalton can be used (e.g. Spectrapor). For diafiltration, ultrafiltration membranes with cut-off in the range of 3-10 kD, preferably 5-10 kD, can be used. Suitable membrane materials include polysulfone and polyethersulfone membranes with or without surface modifications, and, preferably, cellulose or cellulose acetate membranes and their derivatives. Suitable devices include hollow fibre systems with straight or coiled fibres, cassette systems without a screen (open channel), and, most preferably, cassette systems equipped with screens of different types of design. For example, a Hydrosart 10 kD screen channel membrane (Sartorius, Göttingen, Germany) can be used.

After the Interleukin-4 is solubilized and chemically derivatized as described above, the molecule can be further purified prior to renaturation by immobilized metal chelate affinity chromatography (IMAC), by affinity chromatography on an immobilized synthetic ligand or by mid pressure reversed phase chromatography (RP-MPLC). These chromatographic steps can be used alternatively to the cross-flow ultrafiltration step which is described above.

In case of IMAC, the solubilized Interleukin-4 inclusion body (see above) is diluted with buffer to achieve a final guanidinium hydrochloride concentration of not less than 3 M. Below 3 M, the non-native Interleukin-4 molecules tend to precipitate. The preferred buffer for solubilization and further purification by IMAC is e.g. phosphate, but other non-chelating components (so-called non-GOODs buffers) buffering within the preferred pH range of 6-9, most preferably pH 7-8, can also be used instead like e.g. glycine-NaOH, N-ethylmorpholino-HCl or 2-amino-2-methyl-1,3-propandiol-HCl.

IMAC can be performed in both modes of operation, packed-bed or expanded-bed (STREAMLINE-IMAC). Although native Interleukin-4 contains several histidine-residues in α-helical (His(59)-X-X-X-Asp), β-strand (His(1)-X-Cys, His(75)-X-His) and β-turn (His(1)-X-X-Asp) motives, and it was known from the literature that native Interleukin-4 is bound to IMAC (Naveh et al. (1991): US Patent 5,034,133, Schering cooperation) it is surprising that guanidin-denatured Interleukin-4 also binds to IMAC-resins. Metal ions suitable for binding of non-native Interleukin-4 (4 M guanidinium hydrochloride, phosphate buffer, most preferred pH 7), sorted by decreasing binding strength, are Cu>Ni>Co>Zn. Preferably, Ni-ions are used to selectively bind non-native Interleukin-4. The dynamic binding capacity of IMAC-resins is about 10 to 12 g protein/L resin. Expanded-bed mode of operation allows the combination of protein purification and the removal of residual cell wall fragments and other non-proteinicious contaminants. Elution of non-native Interleukin-4 bound to the IMAC-resin charged with appropriate metal-ions can be achieved by a linear or step gradient of a suitable displacer like e.g. histidine, histamine, other literature known displacers for IMAC or, most preferably, by imidazole at concentrations of 10 mM to 1000 mM, preferably 10 to 100 mM, most preferably 10 to 50 mM imidazole. The product containing fractions are combined. Purity of non-native Interleukin-4 after IMAC is generally about 90% as judged by SDS-PAGE analysis (Coomassie-stain) and the average recovery of Interleukin-4 is better than 80%.

Subseqeuntly, Interleukin-4 is renatured to form its biologically active conformation. There are several pathways to refold Interleukin-4 and to form the correct disulfide bridges:

### 1. Matrix-assisted refolding

Renaturation of proteins bound to a chromatography resin is an established method (Las et al. (1984): US5739281, pub. date 14/04/1998; WO9418227, pub. date 18/08/1994, Denzyme, Denmark). A major disadvantage of commonly used ion exchange matrix-assisted refolding is the multi-point attachment of the protein to the resin resulting in a low flexibility of the protein, which may interfere with the formation of the correct three-dimensional structure. Moreover, Interleukin-4 precipitates quantitatively on the resin leading to blockage of the column, if the concentration of the chaotrope is varied according to the patent published by Las and coworkers (see above).

Since guanidin-denatured Interleukin-4 was shown to bind to metal chelate resins, which most probably is due to a single-point or omni-point attachment of Interleukin-4 to the resin, IMAC-assisted refolding is possible. After Interleukin-4 is bound to a matrix like e.g. chelating-sepharose FF or, preferably, STREAMLINE-IMAC (both from Pharmacia), a suitable decreasing gradient of guanidinium hydrochloride and a suitable redox-shuffling system are applied to allow Interleukin-4 to refold to its biologically active form. Gradient shapes may be linearly decreasing gradients or, preferably, a decreasing gradient with a sawtooth profile. In the latter type of gradient a linear decrease is followed by a steep, stepwise smaller increase in chaotrope, followed again by a linear decrease. Redox-shuffling systems are suitably e.g. glutathione-based systems, cysteine systems or 2-mercaptoimidazole systems. Other mercaptans tend to interfere with the metal bound on the resin. The ratio of oxidized and reduced forms of the preferred glutathione may vary over a range of 1:50 to 50:1, preferably 40:1.

Using the matrix-assisted refolding system based on IMAC, Interleukin-4 can be refolded to its biologically active conformation as determined by measurement of the affinity of Interleukin-4 to the α-chain of the Interleukin-4 receptor (IL-4Rα) as described by Shen et al. (1996): European Journal of Biochemistry 240(1): 252-261, Wang et al. (1997): Proceedings of the national academy of sciences of the United States 94(5): 1657-1662). The association (kₒₙ: 4.67 E+07 [M⁻¹ s⁻¹]) and dissociation (k_{off}: 3.81 E-11 [M⁻¹]) constants reflecting the binding affinity were comparable to the reference standard material (kₒₙ: 4.14 E+07 [M⁻¹ s⁻¹]; k_{off}: 4.68 E-11 [M⁻¹]).

### 2. Refolding by dialysis or diafiltration

Prior to dialysis or diafiltration against refolding buffer, the solubilized and, preferably, sulfitolyzed inclusion bodies may be diluted with dilution buffer (e.g. 4 M guanidinium hydrochloride, 0.6 M arginine hydrochloride, e.g. 50 mM phosphate buffer, pH 7) in order to adjust the protein concentration to 0.01 g/L to 0.5 g/L, preferably to 0.05 g/L to 0.3 g/L, most preferably to 0.2 g/L to 0.3 g/L. The sulfite protecting group is then removed by the addition of a mercaptane like e.g. β-mercaptoethanol, reduced glutathione, 2-mercaptoimidazole, or, preferably, cysteine. Dialysis is performed using dialysis bags with a cut-off between 3.5 kD and 10 kD. A 2-fold buffer exchange against a 20-fold volume of refolding buffer containing arginine added at 0.1 to 1.5 M, preferably at 0.4 to 1 M. Diafiltration can be used instead of dialysis in case of large-scale refolding. For this purpose, a suitable membrane with a preferred cut-off of 1 to 30 kD, most preferably of 5 to 10 kD, is employed. Ultrafiltration membranes suitable for this process are e.g. polyethersulfone, polysulfone, cellulose and, most preferably, cellulose-acetate derivatives. Diafiltration is performed against 4 volumes of refolding buffer (see above).

Refolding yields concerning soluble protein are generally better than 80%.

### 3. Refolding by dilution

In this case, different modes of operation can be used alternatively:
a) Reduction of denatured Interleukin-4 in the presence of a chaotropic agent
b) Reduction of Interleukin-4 after dilution
c) Reduction of Interleukin-4 while dilution

It was found that the mode of operation defines the final yield of correctly folded Interleukin-4 and the final recovery of total protein. On the other hand, cysteine hydrochloride and cysteine base give approximately the same refolding yields. The concentration of cysteine is preferably between 0.1 mM and 10 mM, more preferably between 0.4 mM and 2 mM. The initial protein concentration is such that the ratio of correctly folded to misfolded conformer recovered will be maximized, as determined by RP-HPLC or BiaCore-assay. The preferred concentration of Interleukin-4, resulting in the maximum yield of correctly folded conformer, is in the range of about 10 mg/L to 1 g/L, preferably between 50 mg/L to 500 mg/L, more preferably between 250 mg/L and 400 mg/L. Method 3c yields the highest amounts of correctly folded Interleukin-4 (generally about 30-35%) and the highest recovery of total protein (generally about 100%).

### 4. Artificial chaperone systems

Artificial chaperone systems have been described for the refolding of a range of enzymes (Gellman et al. (1994): U.S. Patent 5 563 057, filing date Oct. 94, Res. Foundation; Sivakama et al. (1999): FEBS Letts. 443(2): 215-219). A suitable detergent complexing with the unfolded Interleukin-4, a suitable "stripping" agent removing the detergent from the protein-detergent-complex in a second step, and a redox system (see above) have to be chosen at optimal concentrations to achieve refolding of Interleukin-4. Suitable detergents range from ionic, non-ionic to zwitterionic detergents. As mentioned above, Interleukin-4 inclusion bodies can be solubilized effciently in a range of detergents, which can also be employed during refolding with an artificial chaperone system. If guanidinium-salts are used for solubilizing the inclusion bodies, the anionic detergents like, e.g., sodium dodecyl sulfate, can not be used due to precipitation of guanidinium-alkyl salts.

The preferred detergents are cationic and zwitterionic detergents, more preferred are zwitterionic detergents, even more preferred are CHAPS, CHAPSO, Zwittergent 3-8 to Zwittergent 3-16 and, most preferred, Zwittergent 3-14. Preferred cocentrations, which efficiently solubilize non-native Interleukin-4, are located at or above the CMC of the respective detergent. For example, Zwittergent 3-14 (CMC 0.2 to 0.4 mM) solubilizes non-native Interleukin-4 at 0.1 to 10 mM, more preferably at 0.5 to 5 mM, most preferably 1 to 4 mM.

A suitable "stripping" agent is any compound that effect the critical micellar concentration (CMC) of the detergent, thereby influencing the detergent-protein-interactions. Preferably, these compounds display amphiphilic characteristics, thereby exposing hydrophilic and hydrophobic parts in one molecule. Preferably, cyclic dextrins like, e.g., χ- or, preferably, α-, β-cyclodextrin, or linear dextrins like, e.g., dextrin-15, dextrin-20 or, preferably, dextrin-10 can be employed in such an artificial chaperone system.

Additionally, other compounds affecting the aggregation behavior of Interleukin-4 during refolding may be added to the artificial chaperone system. Suitable compounds are preferably salts at concentrations between 10 mM and the solubility limit of the respective salt like, e.g., NaCl, KCl, Na₂SO₄, (NH₄)₂SO₄, MgCl₂, KI, KSCN, guanidinium-salts, urea or its derivatives. Other suitable compounds include amino acids like, e.g., arginine or leucine, betaines or sulfobetaines, poly(ethylene)-glycols or low molecular weight alcohols. In case of the amino acids, betaines and sulfobetaines, the preferred range of concentrations is between 0.05 and 1 M. Poly(ethylene)-glycols may be added at concentrations between 0.1 mM to 10 mM, preferably at 0.5 to 1 mM.

The protein concentration is in the range of 0.01 to 1 g/L, preferably 0.1 to 0.5 g/L.

### 5. Other methods

The refolding is typically carried out at about +0°C-45°C, preferably about 20-40°C, more preferably about 23-37°C, even more preferably about 25°-30°C for at least about one hour.

In case of using the reduced, solubilized Interleukin-4 as a starting material for refolding, a source of oxygen such as air or oxygen gas is entrained in or otherwise introduced into the refolding buffer so as to effect oxidation. The oxygen can be present in the refolding buffer at any point in time, including before Interleukin-4 or any other reagents are added to the refolding buffer.

The amount of oxygen source introduced will depend, e.g., on the type of vessel utilized, the concentration of Interleukin-4, the type of oxygen source, the type and amount of heavy metals present, the type and amount of reducing agent present, if any, and the type and amount of residual chaotropic agent present as well as the pH of the refolding buffer. Generally, the oxygen will be introduced passively (e.g., as air in head space) using an agitator. Alternatively, the air may be introduced through a sparger. The amount of oxygen present in the refolding buffer must be sufficient to allow oxidation of cysteine-residues of Interleukin-4 in preferably about 1 to 48 hours, more preferably about 1 to 24 hours, most preferably about 1 to 18 hours. A higher oxygen sparging rate is required for large scale refolding.

In case of using the sulfitolyzed, solubilized Interleukin-4 as starting material for refolding, a suitable metal chelating agent like, e.g., ethylenediamintetraacetic acid (EDTA), ethyleneglycol-O,O '-bis-(2-aminoethyl)-N,N,N',N'-tetraacetic acid (EGTA), nitriloacetic acid (NTA) or trans-1,2-diamino-cyclohexan-N,N,N',N'-tetraacetic acid (CDTA) may be added to the refolding buffer in order to suppress undesired oxidation reactions. In this case, oxygen supply is not needed for the oxidation of cysteines to form disulfide bridges.

In order to minimize aggregation of Interleukin-4, other compounds affecting the aggregation behavior of Interleukin-4 during refolding may be added to the refolding buffer. Suitable compounds and/or combination of compounds are described in a separate invention (Peters J (2001): European patent application EP 01127373.7, priority date 22 November, 2001).

After Interleukin-4 is refolded, the following procedures, individually or in any combination, are exemplary of suitable purification procedures for obtaining greater purity: fractionation on affinity, metal chelate, ion exchange or hydroxyapatite columns; precipitation; ultrafiltration; reversed-phase low, middle or high pressure chromatography; chromatography on an ion-exchange resin such as S-Sepharose FF, SP-Sepharose FF, CM-Sepharose FF, Source 30 S, Source 15 S, Mono S (Pharmacia), EMD Fractogel S (E. Merck), Hyper-D CM, Hyper-D S (Biosepra), Macroprep High-S (BioRad), Toyopearl 650-S (TosoHaas); chromatography on hydroxyapatite resins such as Ultrogel Hydroxyapatite (Biosepra), ceramic hydroxyapatite (BioRad), hydroxyapatite (Merck); SDS-PAGE; gel filtration using, for example, Sephadex G-75, Superdex 75, Superdex 200 (Pharmacia), Biosec (E. Merck); chromatography on silica-based reversed-phase resins with C4-, C8-, or C18-ligands, e.g., Vydac C8, Vydac C18, YMC C4, Bakerbond C18, Zorbax C18; chromatography on polymer-based reversed phase resins (polystyrene divinyl benzene copolymer) such as Source 15 RPC, Source 30 RPC (Pharmacia), Amberchrome CG-1000, Amberchrome CG-300 HR, Amberchrom CG-300 SD (TosoHaas), small or medium or large pore RPC-resins (Polymer-Labs), PRP-3 (Hamilton).

In a preferred embodiment, the refolding pool is pH adjusted to about 2.5 to 8, preferably 3 to 5, more preferably 3, clarified by depth filtration, and loaded directly onto a low-pressure reversed phase column. Preferably, the loading buffer contains about 0.1% to 1% acid such as hydrochloric acid, sulfuric acid, trifluoracetic acid, acetic acid or, more preferably, phosphoric acid. The elution buffer preferably comprises about 50-100% (v/v), preferably 70-80%, of an alcoholic or polar aprotic solvent and about 0.1% to 1% acid such as hydrochloric acid, sulfuric acid, trifluoracetic acid, acetic acid or, more preferably, phosphoric acid. Preferably, the solvent is methanol, ethanol, isopropanol, 1- or 2-propanol, t-butanol, dimethylsulfoxide, dimethylformamide, N-methylpyrrolidone, tetrahydrofuran, dioxane, or acetonitrile. Even more preferably, the solvent is ethanol.

The column is then washed with a buffer at pH preferably about 3 to remove unbound impurities, and the Interleukin-4 is eluted with a linear gradient or increasing percentage of the elution buffer. More preferably, the gradient consists of a step (20% elution buffer) and a linearly increasing part (20-90% elution buffer).

The pool from either the affinity or IMAC or, preferably, the mid pressure reversed-phase column is adjusted to pH 5.5 to 9, preferably to pH 6 to 8, more preferably to 6.5 to 7.5, depth filtered and applied preferably on a hydroxyapatite column such as Ultrogel hydroxyapatite, conventional hydroxyapatite or, more preferably, ceramic hydroxyapatite. After washing with starting buffer, which preferably is a phosphate buffer, the column is eluted with an increasing phosphate gradient. The elution buffer contains 300 to 1000 mM phosphate, preferably 500 to 800 mM, more preferably 500 mM. The first part of the gradient involves a step elution (18% elution buffer) and the second part a linear increase up to 60% elution buffer. Finally, the column is washed with 100% elution buffer.

In a preferred embodiment of the invention, the pool after mid pressure reversed-phase chromatography is directly applied onto a polymeric cation exchange resin with sulfonic acid ligand or sulfopropyl ligand (e.g. Source S, TosoHaas TSK SP-5PW-HR, Polymer Labs PL-SCX, Biorad Macroprep S, Perkin Elmer Poros HS, Biosepra Hyper D S or equivalent resins). The column is washed with dilute phosphate buffer (pH 3) and product is eluted applying a gradient of sodium chloride (up to 1 M) in the same buffer.

Starting with a partially purified process pool, such as those mentioned before, the mixture of Interleukin-4 and its variants is loaded onto a reversed-phase liquid chromatography column. The loading solvent is preferably a dilute acid such as hydrochloric acid, sulfuric acid, phosphoric acid, acetic acid or, more preferably, trifluoracetic acid. Preferably, the column is packed with a medium having a particle diameter of about 5-40 µm, more preferably about 10-20 µm, and a pore size of about 100-5000Å. In case of silica reversed-phase media, a C4-, C8- or C18-alkyl side chain may be used. More preferably, synthetic reversed-phase media are employed based on polystyrene divinylbenzene copolymers.

The amount of Interleukin-4 loaded onto the column is generally about 0.01 to 40 g protein/L resin, more preferably about 0.02 to 20 g protein/L resin, and most preferably 1 to 12 g protein/L. In the second step of the process herein, the Interleukin-4 is eluted from the column at a pH of about 2 to 7, preferably of about 2 to 4. The elution buffer preferably comprises about 50-100% (v/v), preferably 70-80%, of an alcoholic or apolar, aprotic solvent and about 0.1% to 1% acid such as hydrochloric acid, sulfuric acid, phosphoric acid, acetic acid or, more preferably, trifluoracetic acid. Preferably, the solvent is methanol, ethanol, isopropanol, 1-or 2-propanol, t-butanol, dimethylsulfoxide, dimethylformamide, N-methylpyrrolidone, tetrahydrofuran, dioxane, or acetonitrile. More preferably, the solvent is ethanol.

The column is then washed with a buffer at a preferred pH of 3 to remove unbound impurities, and the Interleukin-4 is eluted with a linear gradient or increasing percentage of the elution buffer. The gradient consists of a step (20% elution buffer) and a linearly increasing part (20-90% elution buffer).

The temperature of the elution is generally about 20° to 60°C, although higher or lower temperatures may be employed. Preferably, the temperature is maintained at about 20° to 30°C.

After the Interleukin-4 is eluted from the column, it is suitably formulated into a pharmaceutical composition as follows. The eluate is pH-adjusted to pH 3 to 8, more preferably to about 3-5 and diafiltered through a suitable ultrafiltration membrane such as polyethersulfone, polysulfone, cellulose and, more preferably, cellulose-acetate and its derivatives. The concentration of protein is adjusted during the final concentration of the retentate volume to about 1 to 60 g/L, preferably about 5 to 20 g/L. The formulation buffer consists of a pharmaceutically acceptable carrier, i.e., one that is non-toxic to recipients at the dosages and concentrations employed and is compatible with other ingredients of the formulation. For example, the formulation does not include oxidizing agents and other compounds that are known to be deleterious to polypeptides. The final formulation may be a liquid or lyophilized solid.

Interleukin-4 to be used for therapeutic administration must be sterile. Sterility is readily accomplished by filtration through sterile filtration membranes (e.g., 0.2 micron cut-off membranes). Therapeutic Interleukin-4 compositions generally are placed into a container having a sterile access port, for example, an intravenous solution gab or vial equipped with a stopper pierceable by a hypodermic injection needle.

Interleukin-4 ordinarily will be stored in single or multi-dose containers, for example, sealed ampoules or vials, as an aqueous solution, or as a lyophilized formulation for reconstitution. As an example for a lyophilized formulation, 5 mL vials are filled with 1 mL of sterile-filtered aqueous Interleukin-4 solution (20 mg/ml), and the resulting mixture is lyophilized using an appropriate lyophilization program. The infusion solution is then prepared by reconstituting the lyophilized Interleukin-4 using bacteriostatic water-for-injection.

The invention will be more fully understood by reference to the following examples, which are intended to illustrate the invention but not to limit its scope. All literature and patent citations are expressly incorporated by reference.

### C. Examples

### Example 1

### Cell disruption by a process using enzymatic and mechanic treatments (high pressure homogenization)

50 g wet *E. coli*-cells containing inclusion bodies of IL-4 are washed in 250 mL disruption buffer (0.1 M Tris-HCI buffer, pH 7.5, 5 mM EDTA) and centrifuged (8000 g, 15 min). The cell pellet is resuspended in 250 mL disruption buffer and 12 mg lysozyme (~1 mg Iysozyme/g cell dry weight) is added and incubated for 30 min at room temperature. The pre-treated suspension is then homogenized in a lab-scale homogenizer (e.g. Stansted, Bran & Lübbe, Manton Gaulin) at a flow rate of 60-70 mL/min and a homogenization pressure of 500 to 1000 bar (3-5 cycles). The disruption effciency is monitored by either means mentioned in the description section. Typically, more than 85% of the cells are disrupted.

### Example 2

### Inclusion body purification by centrifugation and/or cross flow micro filtration

The suspension of disrupted cells ( Example 1) containing released inclusion bodies of IL-4 is centrifuged (8000 g, 15 min) and the inclusion body pellet is resuspended in 250 mL IB washing buffer (0.1 M Tris-HCI buffer, pH 7.5, 5 mM EDTA, 0.1% Zwittergent 3-14). This washing procedure is repeated another three to five times. Washed IB's are harvested by centrifugation (see above).

Addtionally, inclusion bodies containing IL-4 may be washed by cross-flow microfiltration. For this purpose, a suitable cross-flow device equipped with a suitable microfiltration screen channel membrane (e.g. Hydrosart, 0.45 µ, Sartorius AG, Göttingen), wide channel membrane (Sartorius AG, Göttingen; Pall, Eschborn) or a coiled hollow fibre module (Millipore GmbH, Eschborn) can be used. The purified inclusion bodies are >80% pure as judged by SDS-PAGE.

### Example 3

### Solubilization and chemical modification of IL-4

Washed IBs (Example 2) are resuspended in 125 mL solubilization buffer (7 M Guanidin HCI, 5 mM EDTA, 0.2 M Tris-HCI, pH 7). Sulfitolysis of the disulfide bridges is performed by adding 4 g sodium sulfite (240 min incubation at room temperature with stirring) and 1.3 g potassium tetrathionate (30 min incubation at room temperature with stirring). After sulfitolysis, insoluble material is removed by centrifugation (8000 g, 15 min) and excess sulfite and tetrathionate is removed by diafiltration against GuHCl-dialysis buffer (4 M guanidine-HCl, 5 mM EDTA, 50 mM phosphate, pH 7) using a suitable ultrafiltration membrane (e.g. Hydrosart 10 kD, Sartorius, Göttingen).

### Example 4

### Metal-chelate affinity purification of solubilized and chemically modified IL-4

Metal-chelate affinity chromatography (IMAC) of S-sulfonated IL-4 (Example 3) can be performed on a STREAMLINE column (Amersham Biosciences, Sweden) and an appropriate gel (e.g. STREAMLINE-IMAC-gel; Amersham Biosciences, Sweden). Adjust the gel volume to 3 L in the packed mode of operation. Set the maximum pressure to 1 bar. Adjust the flow rates to 150 cm/h in packed mode and 300 cm/h in expanded mode of operation. The chromatography is performed in IMAC-buffer A (4 M guanidin HCI, 50 mM di-sodium hydrogenphosphate, 0.5 M NaCI, pH 7.0). The dynamic binding capacity of the STREAMLINE-IMAC gel is about 10 g protein/L gel. For elution of IL-4, IMAC-buffer B is used (4 M guanidinium HCl, 50 mM sodium dihydrogenphosphate, 0.5 M NaCI, 50 mM imidazole, pH 7.0). Stripping of the nickel ions is performed using IMAC-buffer C (4 M guanidin HCI, 0.5 M NaCI, 50 mM EDTA, pH 7.0). STREAMLINE-IMAC is charged with nickel ions prior to binding of IL-4 (8.5 g/l nickel-acetate tetrahydrate in distilled water). Product containing fractions of the imidazole-pool are combined resulting in a product purity of about 90% as judged by SDS-PAGE analysis (Coomassie stain). The average recovery of IL-4 is generally above 80%.

### Example 5

### Renaturation of chemically modified IL-4

Refolding of sulfitolyzed IL-4 can be performed using different methods:

### 1. Refolding by dialysis

### Lab-scale

Prior to refolding by dialysis against refolding buffer, the solubilized and sulfitolyzed inclusion bodies (Example 3) are diluted with GuHCl-dilution buffer (4 M guanidine-HCl, 0.6 M arginine-HCl, 50 mM phosphate buffer, pH 7) 7.5-fold resulting in a final volume of 1.8 L and a protein concentration of 0.2 to 0.3 g/L. The sulfite protecting group is then removed by adding 1,21 g/L L-cysteine (2.2 g, 30 min, stirring). Renaturation is performed by two-fold dialysis (16 h and 6 h) against 36 L refolding buffer (50 mM phosphate buffer, pH 6.5, 0.6 M arginine-HCl) at 10°C. The volume of the filtered (Sartobran 300 0.22 µ, Sartorius, Göttingen) dialysate is 1.87 L. The refolding yield concerning soluble protein is generally above 80%. A refolding yield of 30-40 mg/L correctly folded IL-4 R121D Y124D is obtained.

### Large-scale

Large scale dialysis can be performed using specially designed stainless-steel tanks. The two tanks (approx. 300 L volume each) were connected via a pump. A second pump delivered the buffer into the buffer-storage tank. The inlet of the tanks was located on the bottom, whereas the outlet was located at the upper side. Two stainless-steel bars within the tanks were used to hang the dialysis bags into the buffer solution floating through the tank. The bulk volume of buffer was stored in a 1200-L stirred tank and pumped continuously through the two tanks. The buffer was kept at 10°C. Temperature and pump performance were monitored on line throughout the 96-hours of renaturation.

The denatured, sulfitolyzed IL-4 (Example 3) is diluted to a final protein concentration of 200 mg/L (Bradford assay). Subsequently, L-Cysteine is added to a final concentration of 15 mM (1.8 g/l, about 180-fold excess compared to cysteine-residues). The protein suspension is filled into dialysis bags (approx. 1 m) which are closed using plastic clips. The dialysis bags are hanged into stainless-steel tanks equipped with in- and outlets for buffer and metal bars to fix the dialysis bags. Refolding is performed by dialysis against the 20-fold volume (1250 L) of the diluted denatured, chemically modified IL-4 using dialysis buffer (120 mM NaCI, 2 mM KCl, 3 mM sodium dihydrogenphosphate, 7 mM disodium hydrogenphosphate, pH 7.1). After 2 days of dialysis, buffer is exchanged and dialysis is continued for additional 48 hours. The refolding yield concerning soluble protein is generally above 80%. A refolding yield of 30-40 mg/L correctly folded IL-4 R121D Y124D is obtained.

### 2. Refolding by diafiltration

Refolding by diafiltration is performed using a 5-10 kD cut-off membrane (e.g. Hydrosart 10 kD, Sartorius, Germany or Omega screen-channel 5 kD, Filtron, Germany). The sulfitolyzed IL-4 (Example 3), dissolved in a 50 mM phosphate or TRIS-HCl buffer (pH 7) containing 4 M Guanidinium hydrocloride (GuHCl), is applied to the diafiltration system. Removal of GuHCl is performed by diafiltration against 6 volumes of refolding buffer containing 50 mM phosphate or TRIS-HCl (pH 7) and 0.6 M L-arginine. The refolding yield concerning soluble protein is generally above 80%. For details concerning wild-type IL-4 and different muteins refer to Domingues et al. (2000): J. Biotechnol. 84: 217-230.

### 3. Refolding by dilution

In this case, different modes of operation can be used alternatively:
a) Reduction of denatured IL-4 and its muteins in the presence of a chaotropic agent, e.g. guanidinium hydrochloride
b) Reduction of IL-4 and its muteins after dilution
c) Reduction of IL-4 and its muteins while dilution

It was found that the mode of operation defines the final yield of correctly folded IL-4 and the final recovery of total protein. On the other hand, L-cysteine hydrochloride and L-cysteine base give approximately the same refolding yields. Method 3c yields the highest amounts of correctly folded IL-4 (generally 10-35% depending on the respective mutein) and the highest recovery of total protein (approx. 100%).

Refolding method 3c is performed by diluting the S-sulfonated IL-4 (see Example 3) into a refolding buffer consisting of 0.6 M L-arginine-HCl, pH 7.5, 0.4-2.4 mM L-cysteine (depending on protein concentration) and 1 mM ETDA to a final protein concentration of 50 to 250 mg/L. The refolding mixture is stirred gently at room temperature and the refolding kinetics are monitored by RP4-HPLC analysis of samples withdrawn at time intervals. After 24 hours, the refolding reaction is complete and further processed.

### 4. Refolding employing artificial chaperones

The S-sulfonated IL-4 prepared as described in Example 1, Example 2, and Example 3 may best be refolded employing artificial chaperones by dilution into the following refolding buffer: 50 mM phosphate, pH 7.5, 1 mM EDTA, 2 mM Zwittergent 3-14, 1.2 mM L-cysteine, 0.2 M L-arginine or 0.4 M L-lysine or 0.4 M sodium or ammonium sulfate. The final protein concentration may be varied between 10 and 1000 mg/L, preferably from 50 to 500 mg/L. The formation of the protein-detergent-complex is allowed to take place for 3 h. Subsequently, β-Cyclodextrin is added to the refolding mixture to give a molar ratio of β-Cyclodextrin/detergent of 6 (12 mM). The refolding reaction is terminated 4 h after β-CD-addition (total refolding time: 7-8 h). The renaturation is monitored by RP4-HPLC as described in Example 9. The total recovery of soluble protein is better than 85%. The overall refolding yield is approx. 18%. A refolding yield of 45 mg/L correctly folded IL-4 R121D Y124D is obtained.

### Example 6

### Concentration of refolded IL-4

The relatively high volume produced during refolding of IL-4 has to be concentrated throughout the further process steps. In principle, this can be achieved by ultrafiltration or by chromatography. For both techniques examples are given in the following sections.

### a) Ultrafiltration/Diafiltration

Concentration of refolded IL-4 is done using a suitable filtration device equipped with an ultrafiltration membrane (cut-off: 5-10 kD) like e.g. Hydrosart 10 kD (Sartorius, Göttingen) or Omega 5 kD (Filtron, Germany). After reduction of volume to a final protein concentration of about 300 mg/L the product solution is then diafiltered against 4 volumes of refolding buffer (see example 5.1). Recovery of protein is generally 90% or higher.

### b) Mid pressure reversed phase chromatography

A column (e.g. 100 mL bed volume) is packed with a polymer-based reversed phase resins like, for example, Source 30 RPC (Amersham Biosciences, Sweden), CG-1000s (TosoHaas, Germany) or another equivalent large pore polymer RPC resin (e.g. Polymer Labs large pore, 10-25µ or 50-75µ particle sizes, up to 4000Å pore size). The column is equilibrated with buffer A (0.1% acid like trifluoroacetic acid or H₃PO₃). After loading the column with the depth filtered (e.g. Seitz Bio 40/20 cascade, quantitative recovery of IL-4) refolding mixture and washing, product is eluted using a linear gradient of ethanol (1 column volume 20-50% of eluent B, 7 column volumes 50-80% of eluent B) or a suitable other organic solvent. Fractions containing product are analyzed by analytical reversed phase HPLC and pooled according to a purity of more than 50% with respect to the correctly folded isoform. The recovery of IL-4 in the main pool is typically as good as 88%. The purity of IL-4 in the main pool is generally >50% as judged by analytical RP4-HPLC.

### Example 7a

### Intermediate chromatographic purification of partially purified IL-4 by ceramic hydroxyapatite

After dilution (1:5 with distilled water) of the main pool made according to Example 6b, adjust the pH to 6.5 (±0.5) with a basic phosphate salt (e.g. K₂HPO₄). Filter the resulting solution through a depth filter (e.g. Seitz Bio40/20) with quantitative recovery of IL-4. If the starting material was prepared according to Example 6a, adjust the conductivity of the solution with 5 mM phosphate buffer (pH 6.5 (±0.5)) to <5 mS/cm. Pump the solution through a column packed with ceramic hydroxyapatite (14 cm Ø x 10.5 cm height; type-I CHA, BioRad, Germany) previously equilibrated with the above phosphate buffer at a flow rate of 200 cm/h. Wash unbound protein through the column with approximately 3 column volumes of 5 mM phosphate buffer (pH 6.5 (±0.5)), until the OD280 signal reaches the base-line. The product is eluted at a flow rate of 200 cm/h by using a linear gradient of phosphate (20 column volumes). Fractions of 1 L are collected and those containing IL-4 are identified by analytical RP4-HPLC and combined. The recovery of IL-4 in the main pool is typically as good as 85%. The purity of IL-4 in the CHA pool is >70% as judged by analytical RP4-HPLC.

### Example 7b

### Intermediate chromatographic purification of partially purified IL-4 by cation exchange chromatography

Adjust the conductivity of the pooled active solution of IL-4 made according to Example 6 with deionized water to <5 mS/cm. Pump the solution through a column packed with Source 15 S (Amersham Biosciences, 15 µ particle size; column dimension: 14 cm Ø x 10 cm height) previously equilibrated with 20 mM phosphate buffer (pH 3) at a flow rate of 200 cm/h. Wash unbound protein through the column with approximately 3 column volumes of 20 mM phosphate buffer (pH 3), until the OD280 signal reaches the base-line. The product is eluted at a flow rate of 200 cm/h by using a linear NaCl gradient (20 column volumes). Fractions of 1 L are collected and those containing IL-4 are identified by analytical RP4-HPLC and combined. The recovery of IL-4 in the main pool is typically as good as 75%. The purity of IL-4 in the cation exchange pool is >70% as judged by analytical RP4-HPLC.

### Example 8

### Final chromatographic purification of IL-4

The active pool of IL-4 from Example 7a or Example 7b is filtered (e.g. Sartobran 300, 0.22 µ) and applied on a Source 15 RPC column (Amersham Biosciences, 15 µm particel size; column dimensions: 3.2 cm x 12 cm) previously equilibrated with HPLC buffer A (0.1% TFA / water). Subsequently, unbound material is eluted (3 column volumes) and a gradient (16% ethanol, 0.1 CV, 16-40% ethanol, 3 CV, 40-64% ethanol, 7 CV) is used to elute IL-4 at a flow rate of 225 cm/. Fractions (10 mL) are diluted appropriately with HPLC buffer A and analyzed for the correctly folded protein using analytical RP4-HPLC. The recovery of IL-4 in the main pool is typically as good as 87%. The purity of IL-4 in the RPC pool is generally >80% as judged by analytical RP4-HPLC.

### Example 9

### Analytical methods

All the assays used to determine the fractions containing correctly folded IL-4 and its muteins are conventional. For UV absorbance at 280 nm measurement of purified IL-4, an A₂₈₀ Optical Density Unit (OD) is equivalent to 1.6 mg by amino acid composition analysis and to 2.0 mg by Lowry's method. Depending on the amino acid replacements, different factors have to be applied for the different muteins. Lowry's method is described by Lowry et al. (1951) J. Biol. Chem. 193: 265-275. Analytical RP4-HPLC is performed on a YMC iso-butyl C4 column (5 µ, 200Å, 4.6 x 250 mm) at a flow rate of 1.0 ml/min. Detection wavelenghth is 210 nm. Buffer A is 0.1% TFA, buffer B is 0.1% TFA with 70% acetonitrile. The gradient is performed as follows: 0-2 min, 40% B; 2-19.5 min, 40%-85% B; 19.5-20 min, 85%-100%B; 20-21 min, 100% B, 21-22 min, 100%-40% B, 22-25 min, 40% B (re-equilibration). Other IL-4 muteins show a slightly different elution behavior. Depending on the amino acid replacements, IL-4 muteins may elute earlier or later compared to wild type IL-4.

### Example 10

### Biochemical characterization of final IL-4 R121D Y124D produced employing process #1

Employing the methods described in examples 1, 2, 3, 4, 5.1, 6a, 7a, and 8, final products of IL-4 R121D Y124D were prepared and characterized biochemically. C-and N-terminal sequence analysis as well as amino acid analysis revealed the identity of the molecule. The average homogeneity expressed as %-PTH-methionine in the first cycle was 94%. Matrix assisted laser desorption spectroscopy (MALDI) was employed to determine the molecular mass. The average molecular mass of the IL-4 R121D Y124D mutein was determined to 14.995 Dalton (STD: ±3, n=7), which is in good agreement with the predicted molecular mass (14.998 Dalton) and the precision of the MALDI method. Reversed phase (C18-resin) high-pressure liquid chromatography (RP18-HPLC) was used as a high resolution method to determine the purity of the final product, which was 90%. Gel permeation chromatography was performed using a Superdex-75 resin (Amersham Biosciences, Sweden) and 0.6 M NaCl in dilute phosphate buffer at neutral pH. The amount of dimers/oligomers was well below 1% and the amount of smaller degradation products was below the detection limit (0.04%). The endotoxin content was determined according to the pharmacopoe LAL assay. The average endotoxin content was 0.24 EU/mg (STD: ±0.34, n=10). The chromosomal DNA-content of the final products was below the detection limit (8 pg DNA/mg protein) of the Threshold DNA assay (Molecular Devices, USA). The full antagonistic activity of IL-4 R121D Y124D preparations derived from process #1 was shown *in vivo* (Shanafelt AB, Forte CP, Kasper JJ, SanchezPescador L, Wetzel M, Gundel R, Greve JM (1998): Proc. Natl. Acad. Sci. USA 95 (16), 9454-9458; Morton M, Harris P, Xu J, Gorina S, Roczniak S, Fitch N, Gundel R (1999): Am. J. Respir. Crit. Care Med. 159 (3. Suppl.): A660 and Harris P, Lindell D, Fitch N, Gundel R (1999): Am. J. Respir. Crit. Care Med. 159 (3. Suppl.): A230) and *in vitro* employing both the receptor binding assay (Shen et al. (1996): Europ. J. Biochem. 240: 252-261, Wang et al. (1997): Proc. Natl. Acad. Sci. US 94: 1657-1662) and the luciferase reporter cell culture assays. The kₒₙ was 4.3 E+07 [M⁻¹ s⁻¹] and the k_{off} was 4.9E-11 [M⁻¹]. The IC50 value of IL-4 R121D Y124D was 6 nM.

### Biochemical characterization of final IL-4 R121D Y124D produced employing process #2

Employing the methods described in examples 1, 2, 3, 5.3c, 6b, 7a, and 8, final products of IL-4 R121D Y124D were also prepared and characterized biochemically. C- and N-terminal sequence analysis as well as amino acid analysis revealed the identity of the molecule. The average homogeneity expressed as %-PTH-methionine in the first cycle was 94.6%. Matrix assisted laser desorption spectroscopy (MALDI) was employed to determine the molecular mass. The average molecular mass of the IL-4 R121D Y124D mutein was determined to 14.999 Dalton (STD: ±6, n=11), which is in good agreement with the predicted molecular mass (14.998 Dalton) and the precision of the MALDI method. Reversed phase (C18-resin) high-pressure liquid chromatography (RP18-HPLC) was used as a high resolution method to determine the purity of the final product, which was 92%. Gel permeation chromatography was performed using a Superdex-75 resin (Amersham Biosciences, Sweden) and 0.6 M NaCl in dilute phosphate buffer at neutral pH. The amount of dimers/oligomers was well below <0.2% and the amount of smaller degradation products was below the detection limit (0.04%). The endotoxin content was determined according to the pharmacopoe LAL assay. The average endotoxin content was 0.03 EU/mg (STD: ±0.013, n=11). The chromosomal DNA-content of the final products was below the detection limit (8 pg DNA/mg protein) of the Threshold DNA assay (Molecular Devices, USA). The antagonistic activity of IL-4 R121D Y124D preparations derived from process #2 was determined by both the receptor binding assay (Shen et al. (1996): Europ. J. Biochem. 240: 252-261, Wang et al. (1997): Proc. Natl. Acad. Sci. US 94: 1657-1662) and the luciferase reporter cell culture assays. The kₒₙ was 4.1 E+07 [M⁻¹ s⁻¹] and the k_{off} was 4.7E-11 [M⁻¹]. The IC50 value of IL-4 R121D Y124D was 4 nM.

### Example 11

### Luciferase reporter cell culture assay

The reporter gene cell line, D2-49 cells, was established by stable introduction of Iµ/Luc. gene into BL-2 cells, Burkitt's lymphoma cell line. The D2-49 cells were cultured in RPMI 1640 medium supplemented with 10 % fetal calf serum (Hyclone, Utah, USA), 100 µg/ml streptomycin (Gibco BRL, New York, USA), 100 IU/ml penicillin (Gibco BRL, New York, USA) and 800 µg/ml G418 (Gibco BRL, New York, USA). The D2-49 cells were subcultured every 3 to 4 days. For induction of luciferase, 100 µl of the cell suspension at a density of 3 × 10⁶ cells/ml was put into a well of a 96-well plate and cultured with or without recombinant human IL-4 for 24 hours unless otherwise stated.

Luciferase level was measured on the basis of principle that mixture of luciferin with ATP produce a glow type luminescent signal in response to luciferase. In short, after a cultivation of D2-49 cells under specified experimental conditions, LucLite™ solution (Packard, Connecticut, USA) was added to cell suspension. Intensity of the produced luminescence was immediately measured by a microplate scintillation and luminescence counter, TOPCount^{TM} (Packard, Connecticut, USA).

LucLite kit (Packard, Connecticut, USA), luciferase (Sigma, Missouri, USA), IL-2 (Genzyme, Cambridge, USA), IL-5 (Pepro Tech EC Ltd., London, UK), IL-6 (Genzyme ), IL-13 (Serotec, Oxford, UK), IFN- (Genzyme) and dimethylsulfoxide (Sigma) were purchased commercially.

### Example 12

### Purification of murine Interleukin-4 Q116D Y119D

360 g wet *E. coli*-cells containing inclusion bodies of mIL-4 Q116D Y119D are washed in 1800 mL disruption buffer (0.1 M Tris-HCI buffer, pH 7.5, 5 mM EDTA) and centrifuged (8000 g, 15 min). The cell pellet is resuspended in 1800 mL disruption buffer and 86 mg lysozyme (~1 mg Iysozyme/g cell dry weight) is added and incubated for 30 min at room temperature. 7.2 g MgCl₂ (x 6 H₂O) is added and dissolved under stirring. Subsequently, 18 µL of Benzonasesuspension (Merck, Darmstadt; 0.25 U/mL) are added and the suspension is stirred for another 30 minutes at room temperature. The suspension is centrifuged at 8000 x g for 15 minutes and the pellet is resuspended in 1800 mL distilled water (osmotic shock). The disruption effciency is monitored by either means mentioned in the description section. Typically, more than 85% of the cells are disrupted.

The released inclusion bodies are further purified by repetitive centrifugation and washing steps (three times) involving centrifugation at 8000 x g for 15 minutes and resuspension in washing buffer (50 mM Tris-HCl, pH 7.5, 5 mM EDTA, 0.1% Zwittergent 3-14). 180 g (wet weight) of washed IBs are resuspended in 180 mL solubilization buffer (7 M Guanidin HCI, 5 mM EDTA, 0.2 M Tris-HCI, pH 7). The total protein content is determined using the BCA-assay (Pierce) to 2.2 g.

Sulfitolysis of the disulfide bridges is performed by adding 4 g sodium sulfite (240 min incubation at room temperature) and 1.3 g potassium tetrathionate (30 min incubation at room temperature). After sulfitolysis, insoluble material is removed by centrifugation (8000 g, 15 min) and excess sulfite and tetrathionate is removed by diafiltration against GuHCl-dialysis buffer (4 M guanidine-HCl, 5 mM EDTA, 50 mM phosphate, pH 7) using a suitable ultrafiltration membrane (e.g. Hydrosart 10 kD, Sartorius, Göttingen). The total protein content is determined using the BCA-assay (Pierce) to 1.6 g (73% step yield). The purity of the S-sulfonated mIL-4 Q116D Y119D was determined by analytical RP4-HPLC to 25% (peak area percent).

Refolding is performed by diluting the S-sulfonated mIL-4 Q116D Y119D (88 mL) into 6.35 L of refolding buffer consisting of 0.6 M L-arginine-HCl, pH 7.5, 0.8 mM L-cysteine (depending on protein concentration) and 1 mM ETDA to a final protein concentration of 250 mg/L. The refolding mixture is stirred gently at room temperature and the refolding kinetic is monitored by RP4-HPLC analysis of samples withdrawn at time intervals. After 24 hours, the refolding reaction is complete and further processed. 300 mg of correctly folded, underivatized mIL-4 Q116D Y119D are formed (19% overall refolding yield) and the total protein recovery is 1.6 g (100% step yield).

The refolding mixture is adjusted to pH 3 by the addition of 25% HCl and filtered through a depth filter (e.g. Seitz Bio40/20) with quantitative recovery. The filtrate is then applied onto a reversed-phase column previously packed with Amberchrom CG-1000s (Tosohaas), which has been equilibrated with 0.1% phosphoric acid. The resin is loaded up to a capacity of 14 g protein/L resin at a flow rate of 150 cm/h. The column is washed with 3 column volumes (CV) 0.1% phosphoric acid. Then, 5 CV of 20% eluent B (0.1% phosphoric acid, 80% ethanol) is washed through the column. Subsequently, a linear gradient of 20% to 90% eluent B in 7 CV is applied. Fractions of 25% of the column volume are collected and analyzed for mIL-4 Q116D Y119D by RP4-HPLC. Those fractions containing product with more than 20% purity are pooled to give the main pool. In total, 288 mg of correctly folded, underivatized mIL-4 Q116D Y119D are found in the main pool (96% step yield).

The main pool is adjusted to pH 6.0 by the addition of K₂HPO₄ (1 M) and depth filtered (e.g. Seitz Bio40/20) with quantitative recovery.

Intermediate purification is performed on a column packed with ceramic hydroxyapatite (Type I, 80 µ particle size, BioRad) previously equilibrated with 5 mM phosphate buffer (pH 6) at a flow rate of 200 cm/h. The column is loaded up to a capacity of 10 g protein/L resin. Unbound protein through the column with approximately 3 column volumes of 5 mM phosphate buffer (pH 6), until the OD280 signal reaches the base-line. The product is eluted at a flow rate of 200 cm/h by using a linear gradient of 0.5 M phosphate (20 column volumes). The fraction size is 25% of the column volume. Fractions containing mIL-4 Q116D Y119D at a purity of >50% (peak area percent) are identified by analytical RP4-HPLC and combined. The purity of mIL-4 Q116D Y119D in the CHA pool is >70% as judged by analytical RP4-HPLC. A step yield of 247 mg of mIL-4 Q116D Y119D is obtained in the main fraction (86%).

The CHA main pool is filtered (e.g. Sartobran 300, 0.22 µ) and applied on a Source 15 RPC column (Amersham Biosciences, 15 µm particel size) previously equilibrated with HPLC buffer A (0.1% TFA / water). Subsequently, unbound material is eluted (3 column volumes) and a gradient (16% ethanol, 0.1 CV, 16-40% ethanol, 3 CV, 40-64% ethanol, 7 CV) is used to elute IL-4 at a flow rate of 225 cm/. Fractions (10 mL) are diluted appropriately with HPLC buffer A and analyzed for the correctly folded protein using analytical RP4-HPLC. The purity of mIL-4 Q116D Y119D in the RPC pool is >84% as judged by analytical RP4-HPLC. A step yield of 225 mg of mIL-4 Q116D Y119D is obtained in the main fraction (91%). The final overall yield of purified mIL-4 Q116D Y119D corresponding to the total initial protein input (2.2 g) is thus 10%.

The final RPC main pool is diluted with water, sterile filtered through a 0.22 µ filter, aliquoted and lyophilized. The product is stored stable at <-18°C for more than 24 month.

Proteinchemical analysis of the final mIL-4 Q116D Y119D preparation was performed employing standard methods as described in the literature. The identity was confirmed by N-terminal sequencing of the first 15 amino acid residues. The purity was 95% of PTH-methionine in the first cycle. Less than 0.5% mis-incorporated norleucine were found by amino acid analysis. In vitro assay of the binding constants of mIL-4 Q116D Y119D (BiaCore) revealed the correct three-dimensional structure (k_{off}: 1,69 x 10⁻³ [s⁻¹], kₒₙ: 4,34 x 10⁶ [M⁻¹ s⁻¹], n = 9; mIL-4 reference: k_{off}: 0,89 x 10⁻³ [s⁻¹], kₒₙ: 7,51 x 10⁶ [M⁻¹ s⁻¹], n=6). The biological functionality of the mIL-4 Q116D Y119D mutein was shown also in vivo (Nelde A, Grunewald S, Bröcker EB, Sebald W, Duschl A (2002): Allergy, in print).

The endotoxin content as determined by the pharmacopoe LAL assay was <0.03 EU/mg protein.

## Claims

1. A method for preparation of Interleukin-4 or muteins of Interleukin-4 by recombinant expression comprising (a) expression in inclusion bodies, (b) disrupting the cells and separating the inclusion bodies, (c) washing inclusion bodies so obtained, (d) solubilizing the inclusion bodies by denaturation (e) renaturating the expression product and (f) purifying the expression product **characterized in that** the inclusion bodies are washed (c) with a buffer containing a detergent which effectively solubilizes lipids bound to the surface of the inclusion body or lipids contained in cell wall fragments. #

2. A method according to claim 1 wherein the detergent for washing the inclusion bodies is a non-ionic detergent, a ionic surfactant or a zwitterionic detergent.

3. A method according to claim 1 wherein the detergent is a zwitterionic detergent selected from the group , CHAPS, CHAPSO,desoxycholate and the zwittergent series (N-alkyl-N,N-dimethyl-3-ammonio-1-propanesulfonate).

4. A method according to any of claims 1 to 3 wherein the washing buffer for the inclusion bodies is a buffer which maintains the pH between 7 and 10.

5. A method according to any of claims 1 to 4 wherein the washing buffer additionally contains a chelating substance.

6. A method according claim 5 wherein the a chelating substance is selected from the group ethylenediamintetraacetic acid (EDTA), ethyleneglycol-O,O'-bis-(2-aminoethyl)-N,N,N',N'-tetraacetic acid (EGTA), nitriloacetic acid (NTA) or trans-1,2-diamino-cyclohexan-N,N,N',N'-tetraacetic acid (CDTA).

7. A method according to claim 1-6 wherein the recombinant protein is Interleukin 4 R121D Y124D.

8. The method according to any one of claims 1 to 7 wherein the renaturation (e) is done by dialysis, diafiltration or dilution optionally in the presence of artificial chaperones.

9. The method according to claim 8 wherein the renaturation (e) is done in the presence of artificial chaperones.

10. The method according to any one of claims 1 to 9 wherein the purification (f) is done by chromatography.
